# EUROPEAN PATENT APPLICATION

(11) **EP 4 230 614 A1**
(43) Date of publication of application: **23.08.2023**
(21) Application number: 22878674.5
(22) Date of filing: 08.07.2022
(51) Int. Cl.: C07C 68/06, B01J 27/24, C07C 69/96

(54) **ASYMMETIC LINEAR CARBONATE AND METHOD FOR PREPARING ASYMMETIC LINEAR CARBONATE**

(30) Priority: 07.10.2021 KR 20210132850
(71) Applicant: Lg Chem, Ltd., Seoul 07336 (KR)
(72) Inventor: PARK, Jun Beom, Daejeon 34122 (KR); LEE, Hyunyoung, Daejeon 34122 (KR); KIM, Hyun Cheol, Daejeon 34122 (KR)
(74) Representative: Goddar, Heinz J.
(86) International application number: PCT/KR2022/009942
(87) International publication number: WO 2023/058851

(57) **Abstract**

The present disclosure relates to a method for preparing an asymmetric linear carbonate, which comprises subjecting two different symmetrical linear carbonates to a transesterification reaction in the presence of a base catalyst having a heterocyclic structure to prepare an asymmetric linear carbonate.

## Description

### FIELD OF THE INVENTION

### CROSS-REFERENCE TO RELATED APPLICATION(S)

This application claims the benefit of Korean Patent Application No. 10-2021-0132850 filed on October 7, 2021 with the Korean Intellectual Property Office, the content of which is incorporated herein by reference in its entirety.

The present disclosure relates to an asymmetric linear carbonate and a method for preparing an asymmetric linear carbonate.

### BACKGROUND OF THE INVENTION

Since the electrolyte solvent of a lithium ion battery must efficiently dissolve lithium ions and move smoothly, high solubility in salts and low viscosity are required. Therefore, a cyclic carbonate (ethylene carbonate, propylene carbonate, etc.) that efficiently dissolves salts and a linear carbonate (ethylmethyl carbonate, dimethyl carbonate, diethyl carbonate, etc.) having a low viscosity are mixed and used.

In particular, ethyl methyl carbonate (EMC), which is an asymmetric linear carbonate, is the most preferred solvent because it is excellent in energy storage density, charge capacity, charge/discharge recovery, stability, etc. as compared with other solvents.

As a method for preparing such ethyl methyl carbonate, there is a method that utilizes an ester reaction of an alkyl chloroformate with an alcohol in the presence of a basic catalyst, but this method has a problem that the ester reaction is very vigorous, and a highly toxic compound such as phosgene and bisphenol-A must be used as a starting material.

In order to compensate for such a problem, there is a method that utilizes a transesterification reaction between a symmetrical linear carbonate and an alcohol, but the final target compound from the reaction product containing three types of linear carbonates and two types of alcohols has a problem that an asymmetric linear carbonate such as ethyl methyl carbonate must be separated and purified by a complicated process.

Therefore, there is a need to develop a method for producing ethyl methyl carbonate having highly added value so as to eventually facilitate its separation and purification.

### SUMMARY OF THE INVENTION

### TECHNICAL PROBLEM

It is an object of the present disclosure to provide a method for preparing an asymmetric linear carbonate in which a transesterification process can be carried out by a solvent-free process, and thus, only three types of linear carbonates are included in the final reaction product, thus making it easier to separate and purify an asymmetric linear carbonate having highly added value, and a high-purity asymmetric linear carbonate prepared by such a preparation method.

### TECHNICAL SOLUTION

Provided herein is a method for preparing an asymmetric linear carbonate, which comprises subjecting two different symmetrical linear carbonates to a transesterification reaction in the presence of a base catalyst having a heterocyclic structure to prepare an asymmetric linear carbonate.

Also provided herein is an asymmetric linear carbonate, comprising a base compound having a heterocyclic structure.

Hereinafter, the method for preparing an asymmetric linear carbonate according to specific embodiments of the present disclosure will be described in more detail.

However, it will be apparent to those skilled in the art that this is presented as an example of the invention and the scope of the invention is not limited thereby, and that various modifications can be made to the embodiments without departing from the scope of the invention.

Unless explicitly stated herein, the technical terms is for the purpose of describing specific embodiments only and is not intended to limit the scope of the invention.

The singular forms "a," "an" and "the" are intended to include plural forms, unless the context clearly indicates otherwise.

It should be understood that the terms "comprise," "include", "have", etc. are used herein to specify the presence of stated features, regions, integers, steps, actions, elements and/or components, but do not preclude the presence or addition of one or more other features, regions, integers, steps, actions, elements, components and/or groups.

Further, the steps constituting the preparation method described herein are not construed as being limited to the order in which one step and the other steps constituting one preparation method are described herein, unless explicitly stated as being sequential or continuous order or otherwise specified. Therefore, the order of the constituent steps of the preparation method can be changed within a range that can be easily understood by those skilled in the art, and in this case, changes apparent to those skilled in the art accompanying therewith are included in the scope of the invention.

As used herein, the term "substituted or unsubstituted" means being unsubstituted or substituted with one or more substituents selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heterocyclic group containing at least one of N, O and S atoms, or being unsubstituted or substituted with a substituent to which two or more substituents of the above-exemplified substituents are connected. For example, "a substituent in which two or more substituents are connected" may be a biphenyl group. Namely, a biphenyl group may be an aryl group, or it may be interpreted as a substituent in which two phenyl groups are connected.

According to one embodiment of the invention, there is provided a method for preparing an asymmetric linear carbonate, which comprises subjecting two different types of symmetrical linear carbonates to a transesterification reaction in the presence of a base catalyst having a heterocyclic structure to prepare an asymmetric linear carbonate.

The present inventors conducted intensive research on a method for preparing a symmetric linear carbonate such as ethyl methyl carbonate, and confirmed through experiments that when an asymmetric linear carbonate is prepared by subjecting two different types of symmetrical linear carbonates to a transesterification reaction in the presence of a base catalyst having a heterocyclic structure, the transesterification reaction can be carried out in a solvent-free process, and only three types of linear carbonates including two different types of symmetric linear carbonates and asymmetric linear carbonates are contained in the final reaction product, so that the separation and purification of the asymmetric linear carbonate is facilitated, thereby completing the present disclosure.

In the case of a conventional process for preparing an asymmetric linear carbonate, a metal salt alkoxide-based catalyst is used in the transesterification reaction process. A solvent is essentially included for dissolving such a metal salt alkoxide catalyst, which may cause a problem that the final product contains one or more alcohol solvents in addition to the three types of linear carbonates, and thus, the asymmetric linear carbonate, which is the final target compound, must be separated and purified by a complicated process.

However, the base catalyst having a heterocyclic structure used in the method for preparing an asymmetric linear carbonate according to one embodiment has a very high solubility, and thus does not require a separate solvent, and the reaction can proceed in a solvent-free process, whereby the asymmetric linear carbonate can be easily separated and purified from the reaction product containing only three types of linear carbonates.

Specifically, the method for preparing an asymmetric linear carbonate according to one embodiment can prepare an asymmetric linear carbonate by subjecting the two different types of symmetric linear carbonates to a transesterification reaction.

The two different types of symmetric linear carbonates may be two types selected from the group consisting of dimethyl carbonate, diethyl carbonate, dipropyl carbonate and dibutyl carbonate, and for example, it may be dimethyl carbonate and diethyl carbonate.

Further, the asymmetric linear carbonate may be ethyl methyl carbonate, methyl propyl carbonate, ethyl propyl carbonate, butyl methyl carbonate, butyl ethyl carbonate, or butyl propyl carbonate.

Further, as the transesterification reaction is carried out in the presence of the base catalyst having a heterocyclic structure, it may proceed by a solvent-free process. Thereby, no solvent is included in the final reaction product, and the two different types of symmetrical linear carbonates and the reaction product contain only asymmetrical linear carbonates, so that separation and purification of the asymmetric linear carbonate from this final reaction product can be easily carried out.

The base catalyst having a heterocyclic structure may be represented by the following Chemical Formula 1. 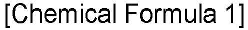 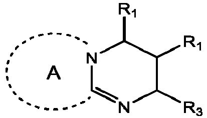 wherein, in Chemical Formula 1,
A is a substituted or unsubstituted alicyclic ring having 1 to 50 carbon atoms; a substituted or unsubstituted alicyclic hetero ring having 1 to 50 carbon atoms; or a substituted or unsubstituted aromatic heterocyclic ring having 1 to 50 carbon atoms.

Further, A may be an alicyclic heterocyclic ring having 2 to 10 carbon atoms that is unsubstituted or substituted with an alkyl group having 1 to 10 carbon atoms.

Alternatively, A may be an alicyclic heterocyclic ring having 2 to 6 carbon atoms that is unsubstituted or substituted with a methyl group.

The alicyclic heterocyclic ring and the aromatic heterocyclic ring may each independently include 1, 2 or 3 heteroatoms selected among N, O, P or S.

Further, R₁ to R₃ may be each independently hydrogen, deuterium, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkoxy group having 1 to 30 carbon atoms, or a substituted or unsubstituted alkylamine group having 1 to 30 carbon atoms.

Further, R₁ to R₃ may be hydrogen or deuterium.

An example of the base catalyst having a heterocyclic structure may be any one selected from the following compounds.

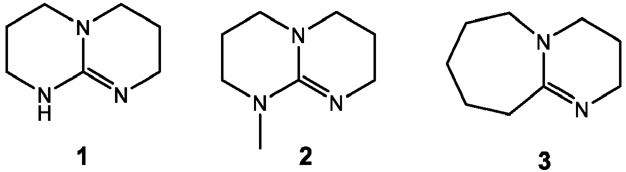

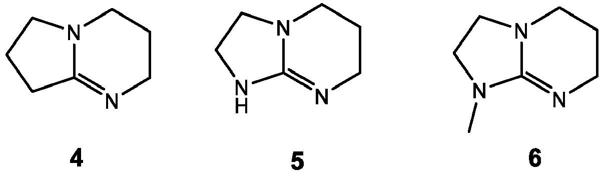

The compound 1 is 1,5,7-triazabicyclo[4.4.0]dec-5-ene (TBD), the compound 2 is 7-methyl-1,5,7-triazabicyclo[4.4.0]dec-5-ene, the compound 3 is 1,8-diazabicyclo[5.4.0]undec-7-ene, the compound 4 is 1,5-diazabicyclo[4.3.0]non-5-ene, the compound 5 is 1,2,3,5,6,7-hexahydroimidazo[1,2-a]pyrimidine, and the compound 6 is 1 -methyl-1,2,3,5,6,7-hexahydroimidazo[1,2-a]pyrimidine.

The base catalyst having a heterocyclic structure may be contained in an amount of 0.1 parts by weight or more, 0.2 parts by weight or more, 0.3 parts by weight or more, 0.5 parts by weight or more, and 10.0 parts by weight or less, 8.0 parts by weight or less, 6.0 parts by weight or less, 5.0 parts by weight or less based on 100 parts by weight of the two different types of symmetrical linear carbonates.

Even if the content of the base catalyst having a heterocyclic structure exceeds 10.0 parts by weight, the reaction is not additionally activated, which is thus uneconomical and inefficient. If the content of the base catalyst having a heterocyclic structure is too small, the transesterification reaction rate may be reduced.

Meanwhile, in the method for preparing the asymmetric linear carbonate, the two different types of the symmetric linear carbonate may be dimethyl carbonate and diethyl carbonate.

Further, the molar ratio of dimethyl carbonate and diethyl carbonate may be 1:0.5 to 1:1.5, 1:0.7 to 1:1.3, and 1:0.9 to 1:1.1. When the content of diethyl carbonate compared to the dimethyl carbonate is too small or too large, the conversion to the final prepared ethyl methyl carbonate may be significantly less.

In the method for preparing an asymmetric linear carbonate of one embodiment, the transesterification reaction may be carried out at a temperature of 90 °C or more, 100 °C or more, 105 °C or more, 110 °C or more, or may be carried out at a temperature of 130 °C or less, 120 °C or less, 110 °C or less.

Further, the transesterification reaction may be carried out for 4 hours or more, 5 hours or more, 6 hours or more, or may be carried out for 12 hours or less, 11 hours or less, 10 hours or less, 8 hours or less.

Further, the transesterification reaction is carried out under pressure conditions of 1 atm or more, 2 atm or more, 3 atm or more, or may be performed under pressure conditions of 10 atmospheres or less, 9 atmospheres or less, 8 atmospheres or less, and 7 atmospheres or less.

Further, the method for preparing an asymmetric linear carbonate according to the one embodiment may further include recovering the asymmetric linear carbonate.

After the transesterification reaction is completed, preferably only three types of linear carbonates can be present in the reaction product. The reaction product may include two different types of symmetrical linear carbonates, and an asymmetrical linear carbonate, which is a compound of interest.

Further, the asymmetric linear carbonate may be separated from the reaction product by a conventional atmospheric pressure or reduced pressure distillation method. That is, when the reaction product is distilled under atmospheric pressure or under reduced pressure, distillation begins in the order of a compound having a low boiling point to a compound having a high boiling point, and finally, the high-purity asymmetric linear carbonate can be recovered.

For example, when dimethyl carbonate, diethyl carbonate, ethyl methyl carbonate are contained in the reaction product, the reaction product is separated in the order of dimethyl carbonate (boiling point: 90°C), ethyl methyl carbonate, and diethyl carbonate (boiling point: 127°C) at the time of distillation, and the high-purity ethyl methyl carbonate of 80% or more, 85% or more, 90% or more, or 99.9% or more can be recovered. The dimethyl carbonate and diethyl carbonate separated at this time can be recovered and reused.

According to another embodiment of the present disclosure, there is provided an asymmetric linear carbonate comprising a base compound having a heterocyclic structure.

The base compound having a heterocyclic structure may be represented by the following Chemical Formula 1: 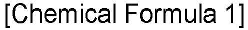 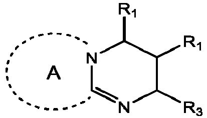 wherein, in Chemical Formula 1,
A is a substituted or unsubstituted alicyclic ring having 1 to 50 carbon atoms; a substituted or unsubstituted alicyclic hetero ring having 1 to 50 carbon atoms; or a substituted or unsubstituted aromatic heterocyclic ring having 1 to 50 carbon atoms.

Further, A may be an alicyclic heterocyclic ring having 2 to 10 carbon atoms that is unsubstituted or substituted with an alkyl group having 1 to 10 carbon atoms.

Alternatively, A may be an alicyclic heterocyclic ring having 2 to 6 carbon atoms that is unsubstituted or substituted with a methyl group.

The alicyclic heterocyclic ring and the aromatic heterocyclic ring may each independently include 1, 2 or 3 heteroatoms selected from N, O, P or S.

Further, R₁ to R₃ may be each independently hydrogen, deuterium, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkoxy group having 1 to 30 carbon atoms, or a substituted or unsubstituted alkylamine group having 1 to 30 carbon atoms.

Further, R₁ to R₃ may be hydrogen or deuterium.

An example of the base compound having a heterocyclic structure may be any one selected from the following compounds.

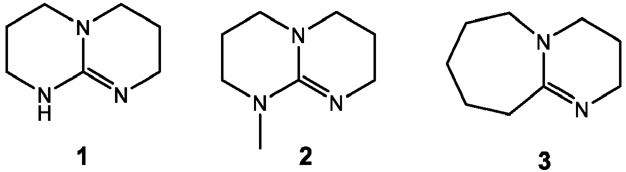

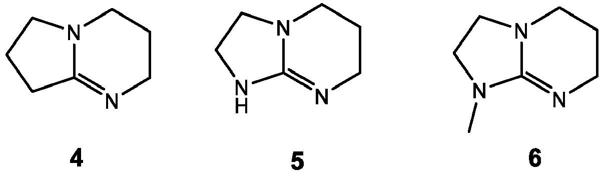

The compound 1 is 1,5,7-triazabicyclo[4.4.0]dec-5-ene (TBD), the compound 2 is 7-methyl-1,5,7-triazabicyclo[4.4.0]dec-5-ene, the compound 3 is 1,8-diazabicyclo[5.4.0]undec-7-ene, the compound 4 is 1,5-diazabicyclo[4.3.0]non-5-ene, the compound 5 is 1,2,3,5,6,7-hexahydroimidazo[1,2-a]pyrimidine, and the compound 6 is 1-methyl-1,2,3,5,6,7-hexahydroimidazo[1,2-a]pyrimidine.

Further, the asymmetric linear carbonate may be ethyl methyl carbonate, methyl propyl carbonate, ethyl propyl carbonate, butyl methyl carbonate, butyl ethyl carbonate or butyl propyl carbonate.

The content of the base compound having a heterocyclic structure may be 0.01 parts by weight or more, 0.05 parts by weight or more, 0.10 parts by weight or more, 0.50 parts by weight or more, and 5.00 parts by weight or less, 3.00 parts by weight or less, and 1.00 parts by weight or less, based on 100 parts by weight of the asymmetric linear carbonate.

### ADVANTAGEOUS EFFECTS

According to the present disclosure, an asymmetric linear carbonate having high purity and highly added value with high conversion can be provided, and also a method for preparing an asymmetric linear carbonate, in which a transesterification reaction is carried out by a solvent-free process, and only three types of linear carbonates are included in the final reaction product, thus making it easier to separate and purify an asymmetric linear carbonate, so that the process control is easy and the mass production is possible.

### DETAILED DESCRIPTION OF THE EMBODIMENTS

Hereinafter, the present disclosure will be explained in detail with reference to the following examples. However, these examples are for illustrative purposes only, and the scope of the present disclosure is not limited thereto.

### Example 1

10 g of dimethyl carbonate (DMC), 13.1 g of diethyl carbonate (DEC), 2 wt% of 1,5,7-triazabicyclo[4.4.0]dec-5ene (TBD) as a catalyst were charged into a 100 mL pressure reactor. At this time, the molar ratio of dimethyl carbonate to diethyl carbonate was 1:1. Then, the temperature of the pressure reactor was raised to 110°C and reacted for 4 hours. At this time, the reaction was carried out under normal pressure in a closed system.

### Example 2

Ethyl methyl carbonate was prepared in the same manner as in Example 1, except that the temperature of the pressure reactor was 120°C.

### Example 3

Ethyl methyl carbonate was prepared in the same manner as in Example 1, except that the temperature of the pressure reactor was 120°C, the charging amount of the catalyst 1,5,7-triazabicyclo[4.4.0]dec-5-ene (TBD) was 4 wt%.

### Comparative Example 1

10 g of dimethyl carbonate (DMC), 5.1 g of ethanol (EtOH), and 1 wt% of sodium ethoxide (NaOEt) as a catalyst were charged into a 100 mL pressure reactor. At this time, the molar ratio of dimethyl carbonate to ethanol was 1:1. Then, the temperature of the pressure reactor was raised to 90 ~ 110°C, and reacted for 4 hours.

### <Experimental Example>

### 1. Gas Chromatograph (GC) Analysis Results

After the transesterification reaction of Examples and Comparative Examples, analysis was performed by gas chromatography (GC) to confirm the products and residues, and the results are shown in Table 1 below.

**[Table 1]**

| | DMC (mol%) | EMC (mol%) | DEC (mol%) | MeOH (mol%) | EtOH (mol%) |
|---|---|---|---|---|---|
| Example 1 | 24.1 | 49.1 | 26.8 | - | - |
| Example 2 | 23.5 | 49.6 | 26.9 | - | - |
| Example 3 | 23.6 | 49.3 | 27.1 | - | - |
| Comparative Example 1 | 22.4 | 22.8 | 5.7 | 38.9 | 10.1 |

Referring to Table 1, it can be predicted that Examples 1 to 3 using TBD as a catalyst do not contain other solvents (methanol, ethanol) other than DMC, EMC, and DEC as a reaction product, and thus EMC can be easily recovered therefrom, whereas it can be predicted that in Comparative Example 1 in which TBD was not used as a catalyst, DMC, EMC, DEC, methanol, and ethanol are all contained as the reaction result, and it is difficult to recover EMC therefrom.

## Claims

1. A method for preparing an asymmetric linear carbonate, which comprises subjecting two different symmetrical linear carbonates to a transesterification reaction in the presence of a base catalyst having a heterocyclic structure to prepare an asymmetric linear carbonate.

2. The method for preparing an asymmetric linear carbonate according to claim 1, wherein:
the two different symmetrical linear carbonates are dimethyl carbonate and diethyl carbonate, and
the asymmetric linear carbonate is ethyl methyl carbonate.

3. The method for preparing an asymmetric linear carbonate according to claim 1, wherein:
the transesterification reaction is carried out in a solvent-free process.

4. The method for preparing an asymmetric linear carbonate according to claim 1, wherein:
the base catalyst having a heterocyclic structure is represented by the following Chemical Formula 1: 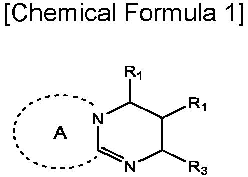 wherein, in Chemical Formula 1,
A is a substituted or unsubstituted alicyclic ring having 1 to 50 carbon atoms; a substituted or unsubstituted alicyclic hetero ring having 1 to 50 carbon atoms; or a substituted or unsubstituted aromatic heterocyclic ring having 1 to 50 carbon atoms, and
R₁ to R₃ are each independently hydrogen, deuterium, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkoxy group having 1 to 30 carbon atoms, or a substituted or unsubstituted alkylamine group having 1 to 30 carbon atoms.

5. The method for preparing an asymmetric linear carbonate according to claim 1, wherein:
the base catalyst having a heterocyclic structure is any one selected from the following compounds:

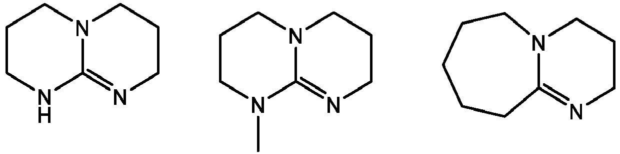

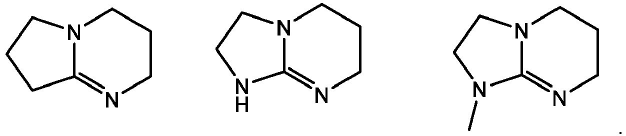

6. The method for preparing an asymmetric linear carbonate according to claim 1, wherein:
the content of the base catalyst having a heterocyclic structure is 0.1 parts by weight or more and 10 parts by weight or less based on 100 parts by weight of the two different types of symmetric linear carbonates.

7. The method for preparing an asymmetric linear carbonate according to claim 1, wherein:
the transesterification reaction is carried out at a temperature of 90°C or more and 130°C or less.

8. The method for preparing an asymmetric linear carbonate according to claim 2, wherein:
a molar ratio of dimethyl carbonate and diethyl carbonate is 1:0.5 to 1:1.5.

9. The method for preparing an asymmetric linear carbonate according to claim 1, further comprising recovering the asymmetric linear carbonate.

10. An asymmetric linear carbonate, comprising a base compound having a heterocyclic structure.

11. The asymmetric linear carbonate according to claim 10, wherein:
the base compound having a heterocyclic structure is represented by the following Chemical Formula 1: 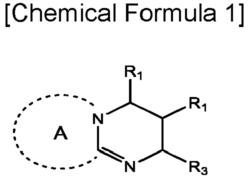 wherein, in Chemical Formula 1,
A is a substituted or unsubstituted alicyclic ring having 1 to 50 carbon atoms; a substituted or unsubstituted alicyclic hetero ring having 1 to 50 carbon atoms; or a substituted or unsubstituted aromatic heterocyclic ring having 1 to 50 carbon atoms, and
R₁ to R₃ are each independently hydrogen, deuterium, a substituted or unsubstituted alkyl group having 1 to 30 carbon atoms, a substituted or unsubstituted alkenyl group having 2 to 30 carbon atoms, a substituted or unsubstituted alkynyl group having 2 to 30 carbon atoms, a substituted or unsubstituted alkoxy group having 1 to 30 carbon atoms, or a substituted or unsubstituted alkylamine group having 1 to 30 carbon atoms.

12. The asymmetric linear carbonate according to claim 10, wherein:
the base compound having a heterocyclic structure is any one selected from the following compounds:

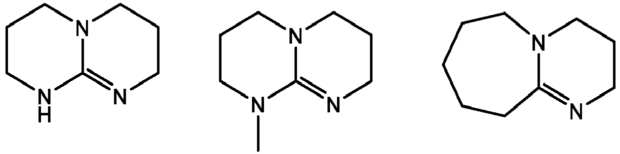

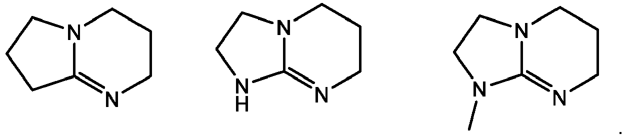

13. The asymmetric linear carbonate according to claim 10, wherein:
the content of the base compound having a heterocyclic structure is 0.01 parts by weight or more and 5 parts by weight or less based on 100 parts by weight of the asymmetric linear carbonate.

14. The asymmetric linear carbonate according to claim 10, wherein:
the asymmetric linear carbonate is ethyl methyl carbonate.
